# EUROPEAN PATENT SPECIFICATION

(11) **EP 4 472 766 B1**
(45) Date of publication and mention of the grant of the patent: **14.01.2026**
(21) Application number: 23712305.4
(22) Date of filing: 03.02.2023
(51) Int. Cl.: G01N 1/22, G01N 1/24, G01N 1/28, G01N 15/06, B01J 20/20, B05B 7/16, B05B 7/24, G01N 21/17, G01N 33/00, A61L 9/14, B01D 51/00, F24F 1/00, G01N 15/14, G01N 15/00, B05B 7/00

(54) **METHOD FOR DETECTING PARTICLES IN ROOM AIR AND CORRESPONDING SYSTEM**
VERFAHREN ZUR DETEKTION VON PARTIKELN IN RAUMLUFT UND ZUGEHÖRIGES SYSTEM
PROCÉDÉ DE DÉTECTION DE PARTICULES DANS DE L'AIR AMBIANT ET SYSTÈME CORRESPONDANT

(30) Priority: 04.02.2022 FI 20225097; 08.07.2022 FI 20225647
(43) Date of publication of application: 11.12.2024
(73) Proprietor: NT Inno Oy, 85800 Haapajärvi (FI)
(72) Inventor: TYTÄRNIEMI, Pasi, 85800 HAAPAJÄRVI (FI)
(74) Representative: Kespat Oy
(86) International application number: PCT/FI2023/050069
(87) International publication number: WO 2023/148430

(56) References cited:
- CN-B- 103 371 139
- CN-U- 209 911 183
- JP-A- 2014 110 857
- US-A1- 2004 005 252
- US-A1- 2011 159 596

## Description

The invention relates to a method for detecting particles in room air, in which method airborne carrier particles are produced. A further object of the invention is a corresponding system.

The known patent publication EP 2239557 A1 of the prior art discloses a method for detecting aerosol particles in the air. This publication also discloses a device that includes an air-sampling unit, an electric humidifier and a fluorescence excitation and detection system. In this method, an air sample is segregated from the room air in the device in which bioaerosol particles of a selected size are conducted onto a polymer layer, where they react with the active molecules. The problem with this method and with the prior art in general is that it is difficult to get particles that are present in the air in small concentrations to enter the device in a sufficient quantity for analysis in order to be able to measure the concentration of the particles in the room air reliably.

Various methods are also known in the prior art for detecting particles in the air that use a marking agent. The marking agent is used to react with a particle to be detected in order to obtain a signal from the particle that can be detected with a measuring device. Analogous marking techniques are disclosed, *inter alia,* in the patent publications CN102061103A and CN103926022B. The measuring device can include different optical sensors with which it is possible to detect a marking agent that has reacted with a particle and thereby identify the particles in the air. The control of the administration of the marking agent to the target space and the transportation of the reacted marking agent to the sensor are, however, problematic.

If harmful or dangerous particles are detected in the air, it is desirable that these particles should be neutralized or that concentrations of substances in the air should be modified based on detection data. A reliable process that can accomplish all this does not exist.

Further prior art can be found in documents JP 2014 110857 A, US 2004/005252 A1, US 2011/159596 A1, and CN 209 911 183 U.

It is an object of the invention to provide an improved method for detecting particles in room air with which it is possible to detect particles more reliably and with greater precision than before. The characteristic features of this invention are indicated in the attached patent claim 1. A further object of the invention is to provide an improved system for detecting particles in the air with which it is possible to detect particles more reliably and with greater precision than before. The characteristic features of this invention are indicated in the attached patent claim 19.

In the method according to the invention for detecting particles in room air, airborne carrier particles are produced, the carrier particles are conveyed into the room air, which room air is circulated, the carrier particles are brought to collide with the particles in the room air, the particles in the room air are brought to bind to the carrier particles, whereby the suspension characteristics of the particles are altered as soon as the particles bind to the carrier particles, the particles are conducted bound to the carrier particles to a sensor together with the air circulating in the room space, the particles are detected by the sensor, and the detection data of the sensor is stored in a memory for further processing.

When the particle to be detected in the air is bound to the carrier particle, the suspension characteristics of the compound are altered so that the particle to be detected can be effectively conducted, bound to the carrier particle, together with the circulating air to the sensor for detection. It is thereby possible for particles in the air to be guided to the sensor, bound to the carrier particles, more effectively and detected with greater precision than before.

Advantageously, the carrier particles take the form of a substance that also has a neutralizing effect on the particles to be detected. It is thus not only possible to detect harmful particles but to reduce their concentration in the room air.

Advantageously, the carrier particles are produced and conveyed into the room air by an air-circulating dispenser by means of which the carrier particles are advantageously conveyed into the upper part of the room air. Circulating air is thus provided in the room space by means of the dispenser. When conveying a carrier particle into the upper part of the room space, the carrier particle and the particle to be detected can be selected in such a manner that the specific weight of their compound is such that the compound descends in the room space, whereby the particle to be detected can be readily conducted, bound to the carrier particle, to the sensor.

Advantageously, the sensor is arranged in an air inlet unit of the dispenser in a lower part of the room space. The particle to be detected bound to the carrier particle can thus descend to a lower part of the room space, from where the particle can be readily conducted together with the circulating air via the dispenser to the sensor for detection.

The sensor can also be arranged in an air outlet vent or in an air outlet duct of the room space. The particles to be detected can thus be conducted bound to the carrier particles together with the natural air circulation of the room space to the sensor.

The airborne carrier particles are produced by means of a method in which a mixture consisting of carrier particles and a liquid carrier agent is formed, which mixture is sprayed onto a hot surface in a process chamber, where the liquid carrier agent is vaporized so as to release the carrier particles into the air of the process chamber, inlet air is fed into the process chamber at a selected temperature in order to cool the free carrier particles to a desired temperature and form a flow of carrier air from an inlet port to an outlet port of the process chamber, and the carrier particles are conducted into the room air. It is thus possible to administer a concentration of carrier particles to be conveyed into the room air with precision.

Advantageously, an active agent is brought to bind to the airborne carrier particles, and a mixture consisting of active agent bound to carrier particles is conveyed into the room air. The detection of particles in the room air can thus be optimized by using an active agent in addition to the carrier particles, wherein both can have an effect that enhances the detection and/or neutralization of particles in room air.

The active agent can take the form of a substance that neutralizes selected particles in room air. It is thus not only possible to detect harmful particles but to reduce their concentration in room air.

The active agent can be a marking agent whose characteristics are altered in the reaction with the particle to be detected, and the particle is detected via the reaction of the marking agent. Particles that cannot be detected directly or with an adequate reliability by a selected sensor can thereby be detected indirectly.

Advantageously, the optical characteristics of the active agent are altered in the reaction with the particle to be detected. It is thus possible to use an optical sensor such as a photoacoustic laser for the detection of the particle.

Two or more different active agents can be administered to room air, wherein a part of the active agents is suitable for detecting selected particles and a part is suitable for neutralizing selected particles. It is thereby possible to continuously monitor and clean the air of a room space simultaneously.

An active agent suitable for neutralizing particles can be brought to bind to the carrier particles as a function of the detected particles. Measures to remove a harmful substance from the room air can thus be initiated immediately based on detection data.

A concentration of selected particles in the room air can be determined by the sensor and an amount of active agent can be administered as a function of the detected concentration of particles. A required amount of active agent can thus be continuously administered to the target space.

Advantageously, a mixture consisting of an active agent bound to carrier particles is produced by means of a method in which a mixture consisting of carrier particles and a liquid carrier agent is formed, which mixture is sprayed onto a hot surface in a process chamber, where the liquid carrier agent is vaporized so as to release the carrier particles into the air of the process chamber, inlet air is fed into the process chamber at a selected temperature in order to cool the free carrier particles to a desired temperature and form a flow of carrier air from an inlet port to an outlet port of the process chamber, an active agent is sprayed into the process chamber, the active agent is brought to collide with the free carrier particles in order to bind the active agent to the carrier particles, and a mixture consisting of active agent bound to carrier particles is conducted into the room air. The concentrations of the carrier particles and of the active agent to be conveyed into the room air can thus be administered with precision.

Advantageously, the particle is identified or recorded as an unknown particle based on an active agent that has reacted with the particle and that is detected by the sensor. A sensor can be taught to identify a harmful particle, although it is also possible for an unknown particle to be detected and recorded as a potential threat.

The carrier particle can be fullerene. Fullerene is a highly unstable carbon molecule which has surprisingly been found to have the required suspension characteristic in air and whose ability to carry substances is easily lost. An active agent, such as helium atoms, can be placed inside the molecular structure of fullerene, or an active agent can be bound to the outside of fullerene. The characteristics of fullerene can also be modified as required.

The carrier particle can be activated carbon. Activated carbon has surprisingly been found to have the required suspension characteristic in air and its ability to carry substances is easily lost.

Advantageously, the active agent is honey or a product processed from honey, such as a concentrate. Honey is known to contain a number of active ingredients due to which honey can act as an antibiotic. It is possible to clean room air by means of the antibacterial effects of honey and the administration of honey to room air can also have health benefits for people located in the room space. The use of honey as an active agent is completely harmless to people located in the room space.

Advantageously, the particle to be detected is mould and/or Actinomycetales. Mould and/or Actinomycetales in a room space is very harmful to people located in the room space as well as to structures of the room space so that it is generally desirable to detect and neutralize mould and/or Actinomycetales located in a room space, such as an office space or a living space, before it causes harm. It has surprisingly been found that activated carbon administered to room air neutralizes mould and/or Actinomycetales by drying them out. In addition, the antibacterial effect of the honey used in combination with activated carbon effectively rids the room air of mould and/or Actinomycetales.

One example of an industrial application in which the method according to the invention can be employed is automated sterilization. Sterilization can be carried out by administering carrier particles suitable for sterilization and a potential active agent into a room space. A waiting period is observed until it is safe for people to enter the sterilized room. With a sensitive and selective sensor, it is possible to detect the concentrations of the carrier particles and of the active agent in the room space during the entire process. The sensor can accordingly also be employed to measure the concentrations of the substances administered into the room space. The method would indicate when the concentrations of the substances are low enough to allow people to enter the room in cases where the carrier particle and/or an active agent employed is harmful to humans. This in turn keeps waiting times to a minimum and is very efficient in spaces in which room downtime is ideally minimized, such as in operating rooms. The method can thus be employed at least in hospitals and cleanrooms.

Another example is the improvement of air quality in a public space, wherein, by means of the administration of a carrier particle and/or of an active agent, salutary substances are diffused in the breathable air, and detection data is applied to monitor air quality and air pollutants in real time so that it is detected if there are changes or if unknown substances enter the indoor air of the public space.

A third example is medical diagnostics, wherein the method according to the invention can be applied in the form of a medication system that makes a diagnosis from the breath of a patient and immediately administers the necessary medication. The taking of the sample in this case can also occur by means of a respiratory mask. Besides the air content of an entire room, room air can thus also designate a more limited volume, such as that of a respiratory mask, or other breathable air.

A system for detecting particles in the air according to the invention, which system is configured to produce airborne carrier particles, includes an air-circulating dispenser configured to administer the airborne carrier particles into the room air, a sensor for detecting particles bound to the airborne carrier particles, and software means for storing the detection data of the sensor in a memory for further processing. Airborne particles can thus be conducted, bound to the carrier particles, to the sensor more effectively and detected with greater precision and more reliably than before.

Advantageously, the sensor is arranged in an air inlet unit of the dispenser or in an air outlet vent of the room. The particle to be detected is thus transported, bound to a carrier particle, together with the air circulating in the room space to the sensor for detection.

Advantageously, the sensor is a photoacoustic laser. A number of different particles can thus be detected directly in the room air once the particles have been conveyed to the sensor.

The dispenser includes a process chamber, a first dispensing reservoir for dispensing a mixture consisting of carrier particles and a liquid carrier agent, a first feed pipe for feeding the mixture of carrier particles and liquid carrier agent from the first dispensing reservoir into the process chamber, a first spraying unit comprising a nozzle for spraying the mixture of carrier particles and liquid carrier agent from the first dispensing reservoir into the process chamber via the first feed pipe, a hot surface in the process chamber for vaporizing the mixture of carrier particles and liquid carrier agent, an outlet port for conducting the carrier particles into the room air, an air inlet unit comprising a motor and a heater for forming a flow of carrier air into the process chamber at a selected temperature, wherein the air inlet unit is connected to the process chamber by an inlet port, and wherein the carrier air flow forms from the inlet port to the outlet port, wherein, in the dispenser, the nozzle of the first spraying unit is oriented in the process chamber towards the hot surface in order to spray the mixture of carrier particles and liquid carrier agent onto the hot surface. The concentration of carrier particles to be conveyed into the room air can thus be administered with precision.

Advantageously, the dispenser is configured to administer an active agent bound to the airborne carrier particles. The detection of particles in room air can thus be optimized by employing an active agent in addition to the carrier particles, wherein both can have an effect that enhances the detection and/or neutralization of particles in room air.

Advantageously, the dispenser includes a process chamber, a first dispensing reservoir for dispensing a mixture consisting of carrier particles and a liquid carrier agent, a second dispensing reservoir for dispensing an active agent, a first feed pipe for feeding the mixture of carrier particles and liquid carrier agent from the first dispensing reservoir into the process chamber, a second feed pipe for feeding the active agent from the second dispensing reservoir into the process chamber, a first spraying unit comprising a nozzle for spraying the mixture of carrier particles and liquid carrier agent from the first dispensing reservoir into the process chamber via the first feed pipe, a second spraying unit comprising a nozzle for spraying the active agent from the second dispensing reservoir into the process chamber via the second feed pipe, a hot surface in the process chamber for vaporizing the mixture of carrier particles and liquid carrier agent, an outlet port for conducting a mixture consisting of active agent bound to carrier particles into the room air, an air inlet unit comprising a motor and a heater for forming a flow of carrier air into the process chamber at a selected temperature, wherein the air inlet unit is connected to the process chamber by an inlet port, and wherein the carrier air flow is formed from the inlet port to the outlet port, wherein, in the dispenser, the nozzle of the first spraying unit is oriented in the process chamber towards the hot surface in order to spray the mixture of carrier particles and liquid carrier agent onto the hot surface, the nozzle of the first spraying unit is closer to the hot surface and to the inlet port than the nozzle of the second spraying unit, and the nozzle of the second spraying unit is closer to the outlet port than the nozzle of the first spraying unit. The concentrations of carrier particles and active agent to be conveyed into the room air can thus be administered with precision.

The invention is illustrated in the following in detail with reference to the attached drawings illustrating embodiments of the invention, wherein
- Figures 1a-1c: schematically illustrate steps for administering carrier particles and an active agent into room air,
- Figure 2: schematically illustrates a cross-section of a dispenser that can be employed in one embodiment in a side view,
- Figure 3a: illustrates a cross-section of a dispenser that can be used in another embodiment in a side view,
- Figure 3b: shows a dispensing unit of a dispenser according to one embodiment,
- Figure 4: schematically illustrates a dispenser according to one embodiment in a top view,
- Figure 5: illustrates an embodiment of the use of a method according to the invention in a room space,
- Figure 6a: illustrates an embodiment for detecting particles in the air using an active agent,
- Figure 6b: illustrates an embodiment for detecting particles in the air without an active agent,
- Figure 7: illustrates an embodiment for neutralizing particles in the air,
- Figure 8: illustrates an embodiment for administering a medication to the lungs.

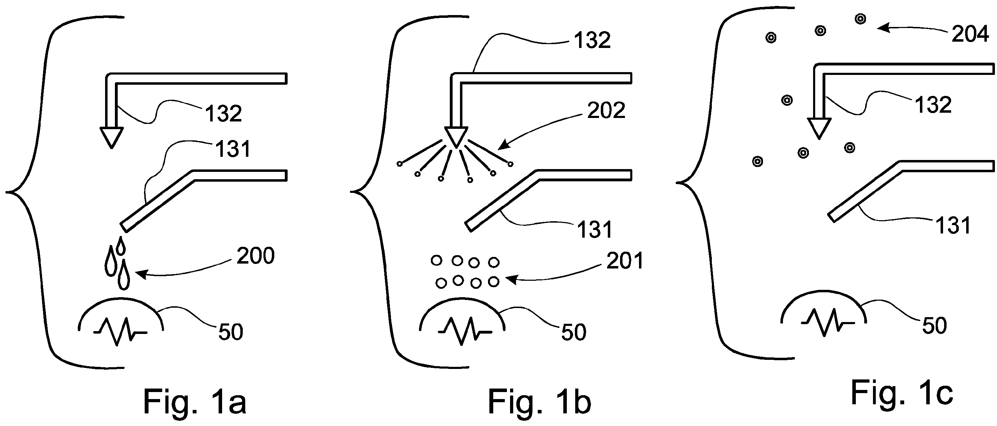

Figures 1a to 1c show steps by means of which an active agent 202 bound to carrier particles 201 can be administered into room air.

In Figure 1a, in a first step, a mixture 200 consisting of carrier particles 201 and a liquid carrier agent is formed and sprayed in a process chamber 60 from a first spraying unit 131 onto a hot surface 50. The mixture 200 of carrier particles 201 and liquid carrier agent vaporizes on the hot surface 50 so that free carrier particles 201 form in the air of the process chamber. The free carrier particles 201 diffuse away from the hot surface 50, the diffusion towards an outlet port 110 being expedited by means of a carrier air flow created in the process chamber 60. The carrier air flow also cools the free carrier particles 201 formed on the hot surface 50, as certain active agents 202 are extremely temperature-sensitive.

In Figure 1b, in a second step, an active agent 202 is sprayed into the process chamber 60 from a second spraying unit 132. The second spraying unit 132 is further away from the hot surface 50 than the first spraying unit 131.

In Figure 1c, in a third step of the method, the active agent 202 collides with the free carrier particles 201, whereby the active agent 202 adheres to the carrier particles 201. The active agent 202 bound to the carrier particles 201 is transported, in the form of a mixture 204 consisting of active agent 202 bound to carrier particles 201, by the carrier air flow to the outlet port 110 of the process chamber 60. The mixture 204 of active agent 202 bound to carrier particles 201 is conducted into the room air from the outlet port 110.

In cases where an active agent 202 is not employed, carrier particles 201 instead being employed on their own for the detection, the administration of the carrier particles 201 into the room air can occur analogously to Figures 1a-1c, although a second spraying unit 132 is not employed.

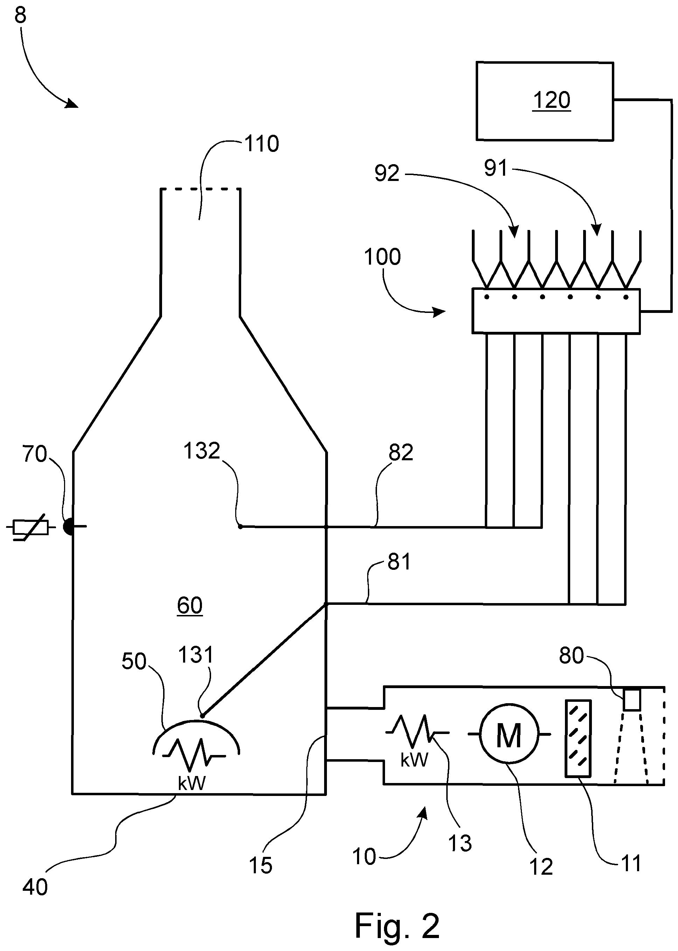

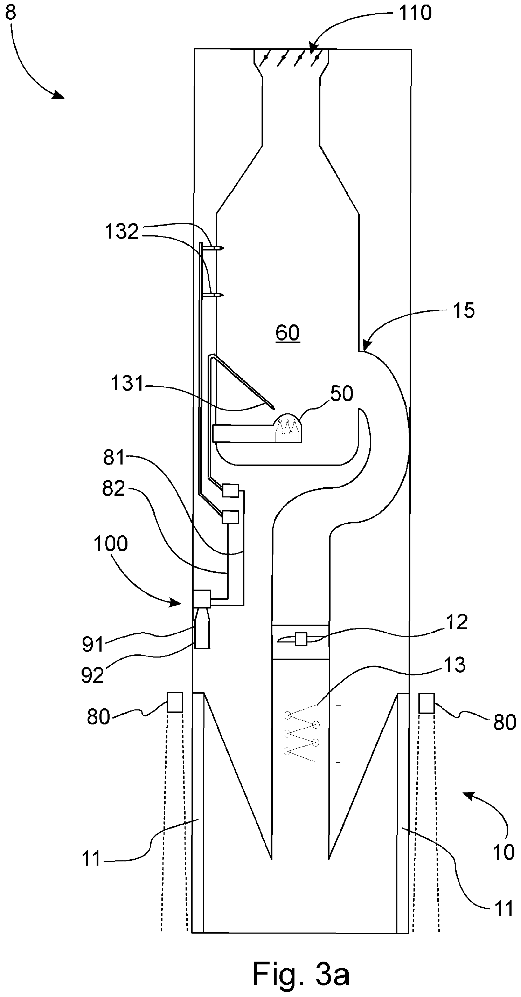

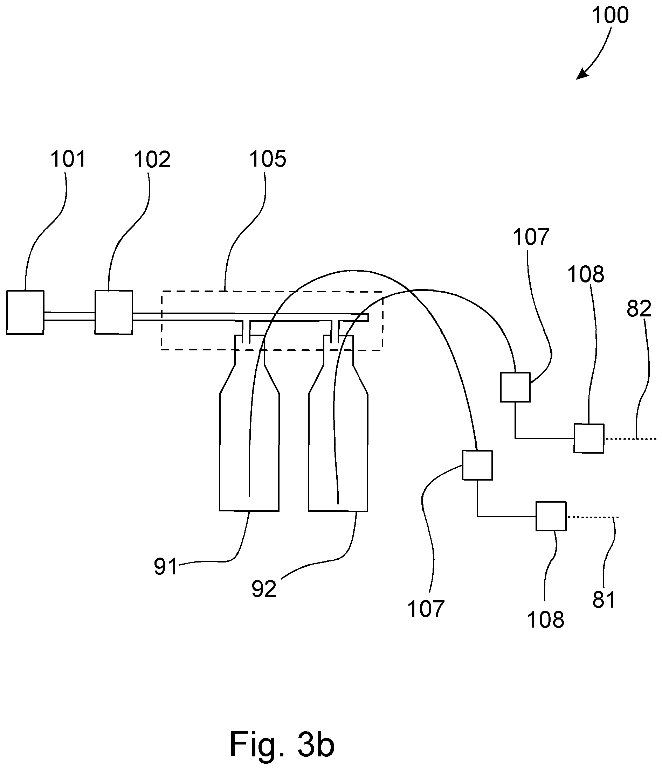

Figure 2 shows an embodiment of a dispenser 8. Figure 3a shows another embodiment of a dispenser 8. Figure 3b shows an embodiment of a dispensing unit 100 of a dispenser 8 in greater detail.

A mixture 200 of carrier particles 201 and a liquid carrier agent is placed in a first dispensing reservoir 91. The mixture 200 of carrier particles 201 and liquid carrier agent can be, for example, a mixture of activated carbon and water. An active agent 202 is placed in a second dispensing reservoir 92. A constant pressure is generated in the first dispensing reservoir 91 and in the second dispensing reservoir 92 by means of an air pump 101 and a pressure regulator 102, which are connected to the first dispensing reservoir 91 and to the second dispensing reservoir 92 via a manifold 105. The mixture 200 of carrier particles 201 and liquid carrier agent flows from the first dispensing reservoir 91 along a first feed pipe 81 at a constant pressure to a flow regulator 107 and onwards via a high-pressure pump 108 into a first spraying unit 131. The active agent 202 flows from the second dispensing reservoir 92 along a second feed pipe 82 at a constant pressure to a flow regulator 107 and onwards via a high-pressure pump 108 to a second spraying unit 132. The first spraying unit 131 and the second spraying unit 132 can comprise, for example, piezo nozzles. The pressure is raised to a level suitable for piezo nozzles by means of the high-pressure pumps 108.

The process chamber 60 contains a hot surface 50, which is heated to 70-300 °C by means of a high-power heating resistor prior to the spraying of the mixture 200 of carrier particles 201 and liquid carrier agent into the process chamber 60. The mixture 200 of carrier particles 201 and liquid carrier agent sprayed from the first spraying unit 131 into the process chamber 60 vaporizes on the hot surface 50, whereby the carrier particles 201 are released into the process chamber 60. The free carrier particles 201 are cooled to a desired temperature by means of inlet air. Regulation of the temperature is essential in light of the temperature sensitivity of the active agents 202 sprayed into the process chamber 60.

An air inlet unit 10 is arranged in an inlet port 15 of the dispenser 8, the motor 12 of the air inlet unit 10 sucking air from outside the dispenser 8 into the interior of the dispenser 8 through an air filter 11. A carrier air flow of 0.5-30 l/s, preferably 1-10 l/s, from the inlet port 15 to the outlet port 110 is produced in the process chamber 60 by means of the air inlet unit 10. The carrier air flow transports the free carrier particles 201 from the hot surface 50 towards the outlet port 110. In the air inlet unit 10, air passes through a heater 13 into the process chamber 60. The inlet air can thus be cleaned and heated to an appropriate temperature, for example 10-60°C, because certain active agents 202 are very temperature-sensitive. In addition, the blowing of the air inlet unit 10 produces an overpressure in the process chamber 60 in relation to the ambient room air, which improves the binding of the active agents 202 to the carrier particles 201. The heating of the hot surface 50 and the reduction of the size of the outlet port 110 in relation to the inlet port 15 also raise the pressure in the process chamber 60. The pressure in the process chamber 60 of the dispenser 8 in operation is typically 5-150 Pa, preferably 30-100 Pa, relative to the air pressure of the ambient room air.

The dispenser 8 illustrated in the figure can also be employed without an active agent 202 for the administration of only carrier particles 201 into the room air.

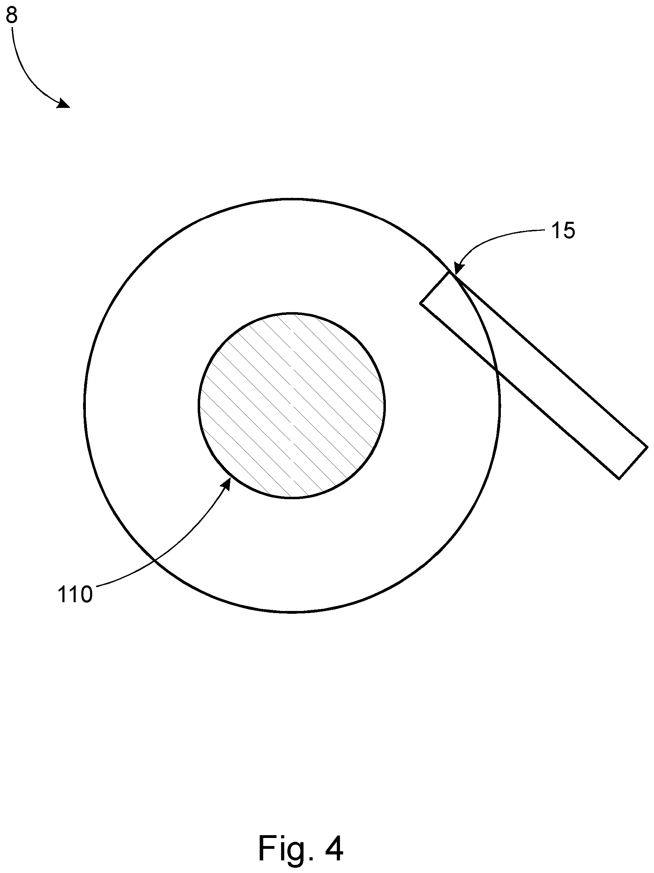

The top view of an embodiment of a dispenser 8 shown in Figure 4 illustrates the geometry of the air inlet unit 10 and the process chamber 60. The inlet port 15 is joined to the outer edge of the process chamber 60 tangentially. The blowing of the inlet air thereby provides a rotation and mixing of the air mass in the process chamber 60.

The active agent 202 is sprayed into the process chamber 60 from the second spraying unit 132, which is located further away from the hot surface 50 than the first spraying unit 131. The distance between the nozzle of the first spraying unit 131 and the nozzle of the second spraying unit 132 can be, for example, approximately 20 cm. The active agent 202 can be mixed, e.g., with water so that the active agent 202 is easy to spray into the process chamber 60. For example, honey or a product made from honey, such as a concentrate, can be employed as the active agent 202. The active agent 202 is sprayed into the cooled carrier particles 201, whereby the active agent 202 binds to the carrier particles 201. The mixture 204 of active agent 202 bound to carrier particles 201 is conducted together with the flow of carrier air to the outlet port 110 of the dispenser 8 and from there into the room air, either into the air volume of an entire room or into a more limited volume such as that of a respiratory mask.

The dispenser 8 further includes a control unit 120 by means of which the spraying of the mixture 200 of carrier particles 201 and liquid carrier agent into the process chamber 60, the spraying of active agent 202 into the process chamber 60, the heating of the hot surface 50, the heater 13 and the motor 12 of the air inlet unit 10 are controlled.

The bottom or a side of the process chamber 60 includes a maintenance hatch 40 for maintenance.

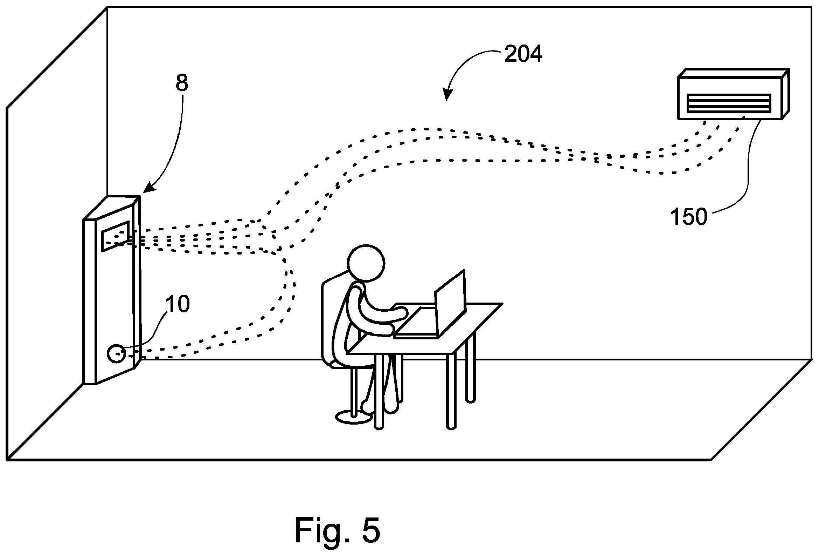

Figure 5 shows one embodiment of the use of a dispenser 8 according to the invention. The dispenser 8 can be arranged, for example, in an office space in order to administer an active agent 202 in the form of a mixture 204 of active agent 202 bound to carrier particles 201 into the room air. The active substance 202 can be, for example, a medication that is administered to people's lungs via the room air. Analogously, it is possible in the embodiment illustrated in Figure 5 to employ only carrier particles 201 in order to monitor and/or clean the room space.

The method according to the invention can be implemented, for example, by means of any of the dispensers 8 described in the foregoing. The dispenser 8 can be based on, for example, the device disclosed in Finnish patent application no. 20206372. The method can also be implemented by some alternative means by means of which airborne carrier particles can be produced and/or an active agent can be conveyed, bound to carrier particles, into air in a selected manner. The dispenser 8 described in the foregoing can also be part of a system according to the invention.

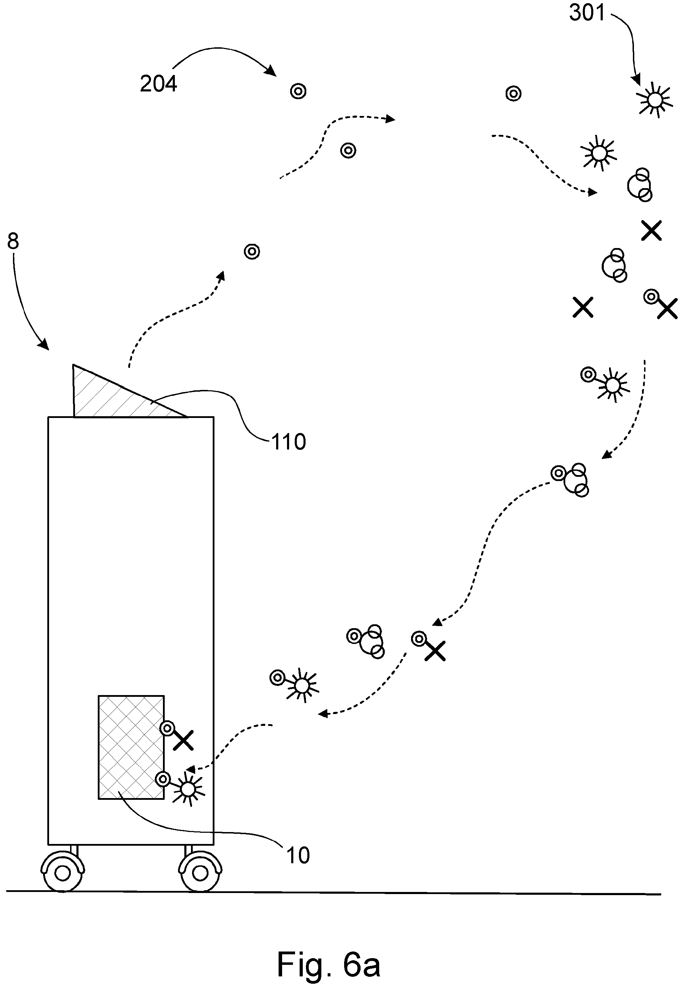

Figure 6a shows a method according to the invention for detecting particles 301 in the air. The method can be employed to detect, for example, viruses, mould or Actinomycetales in the air. Viruses can enter the air, for example, from the exhaled breath or clothing of a person in the room. Mould and Actinomycetales, for their part, can appear in building structures from where they get into the room air. For the detection, it is possible to use, for example, activated carbon or fullerene as the carrier particle 201 while the active agent 202 is a selected marking agent by means of which a virus can be detected and identified. An active agent 202 can be brought to bind to the inside or to the outside of fullerene. Especially when detecting and neutralizing mould and/or Actinomycetales in room air, it is possible to use activated carbon as the carrier particle 201 and honey as the active agent 202. Both activated carbon and honey alone have a neutralizing effect on mould and Actinomycetales. The use of an airborne carrier particle 201 is a prerequisite for conveying honey in suspended form into the room air. A synergistic effect of the activated carbon and the honey is thereby achieved that effectively cleans the room air.

The mixture 204 consisting of active agent 202 bound to carrier particles 201 produced by the dispenser 8 is conducted in suspended form into the room air. The particles 301 to be detected in the room air collide with the active agent 202, whereby the characteristics of the active agent 202 are altered in cases where the active agent 202 is a marking agent. For example, the optical characteristics of the active agent 202, such as its colour, can change during the reaction. As the air circulates, the reacted active agent 202 is transported bound to the carrier particle 201 to the sensor 80, which detects the active agent 202 that has reacted with the particle 301. A part of the reacted active agent 202 finally ends up in the air outlet vent 150 of the indoor space and a part is transported to the air inlet unit 10 of the dispenser 8, where a sensor 80 can be arranged. The sensor 80 can be, for example, a photoacoustic laser, which detects active agent 202 that has reacted with a particle 301. The sensor 80 can also be arranged in an air outlet vent 150 or in an air outlet duct of a space, which increases the probability of detecting the particles 301. In addition to the air circulation created by the dispenser 8, a room space typically has a natural air circulation so that part of the carrier particles 201 and active agents 202 administered into the room air invariably end up in the air outlet duct via the air outlet vent 150. The sensor 80 can also be arranged exclusively in the air outlet vent 150 or in the air outlet duct. Therefore, at least one sensor 80 or, where necessary, a plurality of sensors 80 continuously monitor any dangerous substances, such as viruses, present in the air of the space. If dangerous or harmful substances are detected, an alarm is triggered. Detection data is stored in a memory for further processing by software means that can also perform other tasks, such as the real-time display of detection data and the triggering of an alarm. The sensor 80 can be configured to look for a specific known virus or the sensor 80 can also detect an unknown virus or some other unknown particle 301 that can be categorized as dangerous. The particles 301 in the air can also adhere at least partially to the carrier particles 201 and/or to the active agent 202 so that, with the increase in specific weight, the particles 301 end up at the sensor 80 in the air inlet unit 10 of the dispenser 8 close to the surface of the floor (Figure 2, 3a). The sensor 80 can detect particles 301 directly or by means of a marking agent. As illustrated in Figure 6a, the dispenser 8 can blow outlet air towards the ceiling of the room space into the upper part of the room space while the sensor 80 is located in a lower part of the room space in the air inlet unit 10 of the dispenser. The mixture 204 of carrier particles 201 and active agent 202 thus drifts together with the air circulation generated by the dispenser 8 in such a manner that the mixture 204 of carrier particles 201 and active agent 202 dispensed into the upper part of the room space slowly descends to the lower part of the room space and onwards to the sensor located in the air inlet unit 10. In other words, the dispenser 8 circulates the room air from the outlet port 110 to the air inlet unit 10.

The sensor can take the form of, for example, photoacoustic spectroscopy by means of which it is possible to detect, identify, and determine the concentration of, a very wide range of different substances directly from the room air. With photoacoustic spectroscopy, it is possible to detect, for example, formaldehyde in room air, a gas that is harmful to humans in high concentrations, as well as other VOCs and numerous organic and inorganic compounds. The photoacoustic spectroscopy sensor 80 takes a sample from the room air into a closed space. Sampling can occur, for example, in one-minute cycles. Pulsed electromagnetic radiation is fed into the space using a radiation source, for example a scanning laser or a broad-spectrum infrared radiation source, and filters as well as, for example, a rotating chopper for pulsing. The energy absorbed by the gas is detected by a microphone, which detects the variation in gas pressure caused by the radiation source. The wavelength range of the radiation source can be adapted, where appropriate, to a specific substance to be detected, or a wide range of wavelengths can be employed to detect and identify, as well as measure the concentration of, a plurality of different substances. For example, a GASERA ONE PULSE measuring device can be employed as the sensor 80. Although photoacoustic spectroscopy requires a sample to be taken from the room air into a closed space, it is an essential feature of the invention that the method according to the invention allows the entire room space to be monitored, since the particles to be detected can be transported from anywhere in the room space to the sensor 80 by means of the airborne carrier particles.

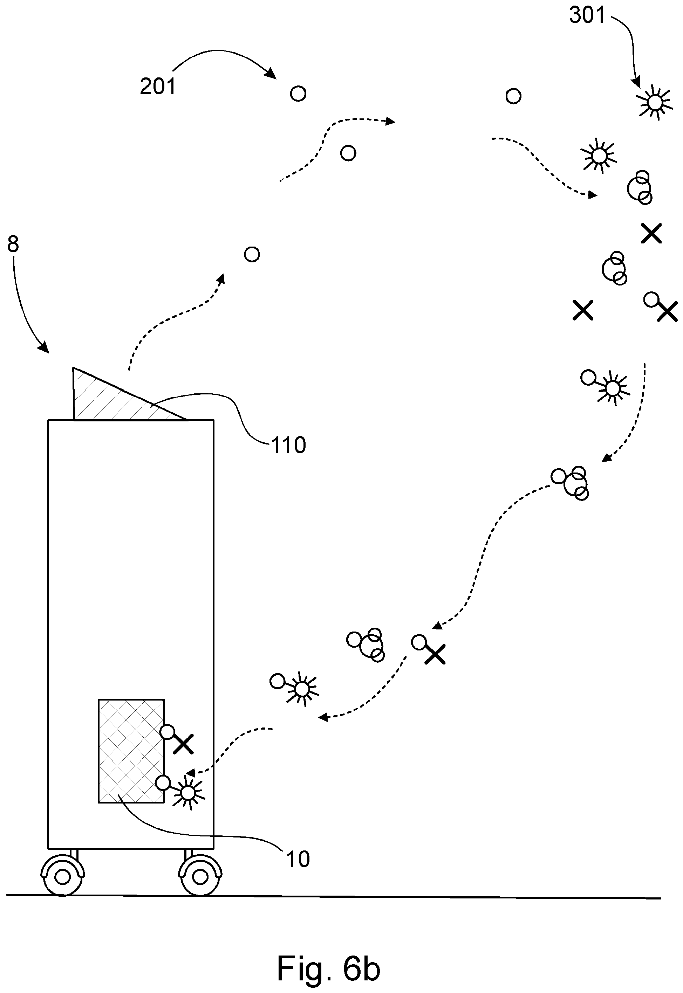

Figure 6b shows another method according to the invention for detecting particles 301 in the air. No active agent is used in this case, but rather solely carrier particles 201 are conveyed into the room air. The air circulation created by the dispenser 8 is adapted in such a manner that the dispenser 8 blows carrier particles 201 from the outlet port 110 into the upper part of the room space towards the ceiling of the room. Both the carrier particles 201 and the particles 301 to be detected in the room air typically have a very low specific weight so that they can remain suspended in the room air for very long periods of time, whereby it is difficult to get the particles 301 to be detected to the fixed sensor 80. In tests, when activated carbon was used as the carrier particle 201, the carrier particles 201 remained suspended in the air for up to 10 minutes, which allowed a very effective binding of the particles 301 to be detected in the air to the carrier particles 201. The binding of an active agent 202 to a carrier particle 201 can increase the specific weight so that the specific weight of the mixture 204 of carrier particles 201 and active agent 202 can be higher and the suspension time in the air thus shorter compared to a carrier particle 201 on its own. The carrier particles 201 are brought to collide with particles 301 suspended in the room air, whereby the particles 301 are brought to bind to a carrier particle 201. The specific weight of a particle 301 bound to carrier particles 201 is thus higher than that of the individual components so that they descend into the lower part of the room space. The particles to be detected can thus be effectively transported to the sensor 80 in the air inlet unit 10 of the dispenser 8 in the lower part of the room space, as illustrated in Figure 6b.

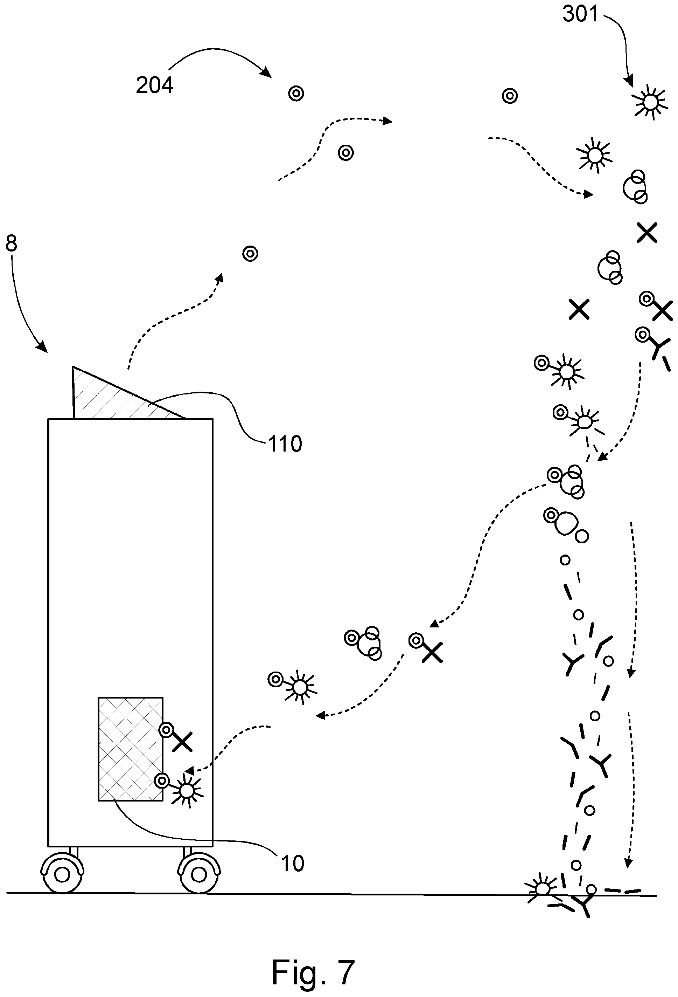

Figure 7 shows a method according to the invention for neutralizing particles 301 in the air. An air filter 11 located in the air inlet unit 10 of the dispenser 8 contributes to the filtering of the circulating air and the removal of impurities from the same. However, the actual neutralization of the particles 301 in the air occurs through the administration of an active agent 202 suitable for the purposes of neutralization, bound to the carrier particles 201, into the air. According to studies, a carbon particle can destroy the structure of a virus. Carbon can also neutralize mould and Actinomycetales through drying. A carbon particle employed as the carrier particle 201 can consequently neutralize a virus, mould and/or Actinomycetales by itself. It is also possible for, for example, parts of a virus to adhere to a carbon particle, which parts are transported to the sensor 80, whereby a carbon particle can also act as a marking agent. It is therefore also possible in the neutralization process illustrated in Figure 7 to only employ the carrier particle 201 without an active agent 202.

The administration of an active agent 202 can be regulated based on the detections performed by the sensor 80. Whenever dangerous or harmful particles 301 are detected by the sensor 80 or the concentration of detected particles 301 changes, the quantity of active agent 202 administered is modified. The active agent 202 administered into the space can be selected as a function of the particles 301 detected. It is also possible to administer a number of different active agents 202 at the same time, wherein, for example, a part is suitable for detecting particles 301 and a part is suitable for neutralizing particles 301. The measurement data collected by the sensor 80 is stored and sent to a database on a server. The collected measurement data can be used to produce real-time graphs and presence maps for detected particles 301.

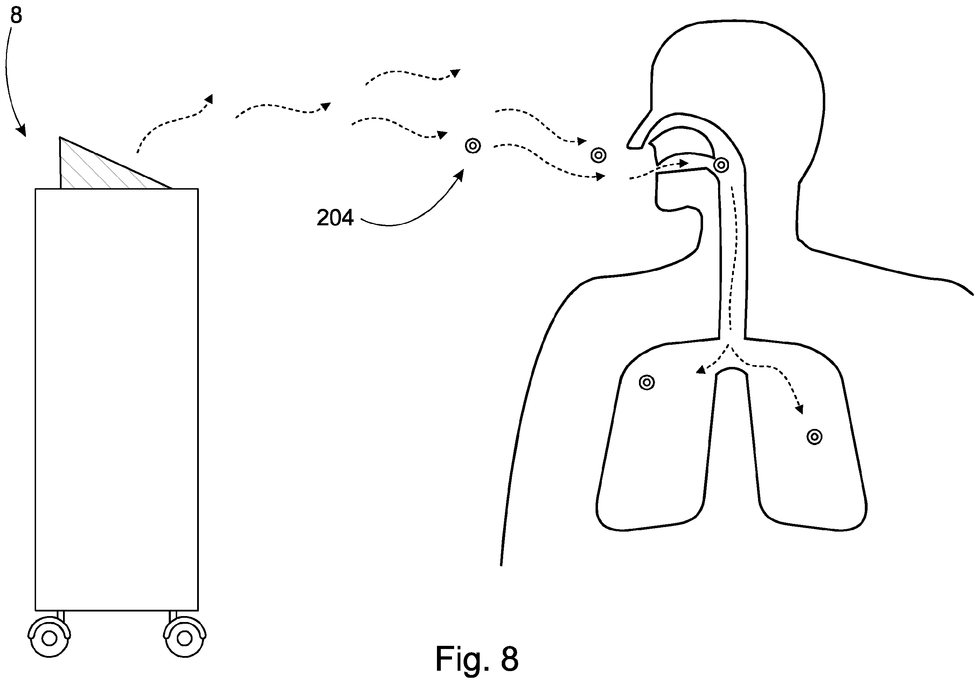

Figure 8 illustrates how a mixture 204 consisting of active agent 202 bound to carrier particles 201 can be employed to strengthen the human immune system against, for example, viruses. A carbon molecule such as fullerene or activated carbon, which can have a salutary effect on the immune system on its own, can be employed as the carrier particle 201. The carrier particles 201 are transported together with the breathable air to the mucous membranes and lungs of the person. The carrier particle 201 stimulates the cellular structure of the lung mucosa, thereby fortifying the cell membrane and the immunity of the cells. In addition to this stimulation, the carrier particle 201 can inhibit the replication of the virus in a cellular structure. A carrier particle 201 can stimulate and strengthen the immune system up to six months after administration. It is simultaneously possible to administer an active agent 202 in the form of a medication to the mucous membranes and lungs together with the carrier particle 201. The active agent 202 can be, for example, a vaccine that protects against a virus.

## Claims

1. A method for detecting particles (301) in room air, in which method
- airborne carrier particles (201) are produced,
- carrier particles (201) are conveyed into the room air, which room air is circulated,
- the carrier particles (201) are brought to collide with the particles (301) in the room air,
- the particles (301) in the room air are brought to bind to the carrier particles (201), whereby the suspension characteristics of the particles (301) are altered as soon as the particles (301) bind to the carrier particles (201),
- the particles (301) are conducted bound to the carrier particles (201) to a sensor (80) together with the air circulating in the room space,
- the particles (301) are detected by the sensor (80), and
- the detection data of the sensor (80) is stored in a memory for further processing,
**characterized in that** the airborne carrier particles (201) are produced by means of a method in which
- a mixture (200) consisting of carrier particles (201) and a liquid carrier agent is formed, which mixture is sprayed onto a hot surface (50) in a process chamber (60), where the liquid carrier is vaporized so as to release the carrier particles (201) into the air of the process chamber (60),
- inlet air is fed into the process chamber (60) at a selected temperature in order to cool the free carrier particles (201) to a desired temperature and form a flow of carrier air from an inlet port (15) to an outlet port (110) of the process chamber (60),
- the carrier particles (201) are conducted into the room air.

2. The method according to claim 1, **characterized in that** the carrier particles (201) take the form of a substance that also has a neutralizing effect on the particles (301) to be detected.

3. The method according to claim 1 or 2, **characterized in that** the carrier particles (201) are produced and conveyed into the room air by an air-circulating dispenser (8) by means of which the carrier particles are advantageously conveyed into the upper part of the room space.

4. The method according to any of claims 1 - 3, **characterized in that** the sensor (80) is arranged in an air inlet unit (10) of the dispenser (8) in a lower part of the room space.

5. The method according to any of claims 1 - 3, **characterized in that** the sensor (80) is arranged in an air outlet vent (150) or in an air outlet duct of the room space.

6. The method according to any of claims 1 - 5, **characterized in that** an active agent (202) is brought to bind to the airborne carrier particles (201), and a mixture (204) consisting of active agent (202) bound to carrier particles (201) is conveyed into the room air.

7. The method according to claim 6, **characterized in that** the active agent (202) takes the form of a substance that neutralizes selected particles (301) in room air.

8. The method according to claim 6 or 7, **characterized in that** the active agent (202) is a marking agent whose characteristics are altered in the reaction with the particle (301) to be detected, and the particle (301) is detected via the reaction of the marking agent.

9. The method according to any of claims 6 - 8, **characterized in that** the optical characteristics of the active agent (202) are altered in the reaction with the particle (301) to be detected.

10. The method according to any of claims 6 - 9, **characterized in that** two or more different active agents (202) are administered to room air, wherein a part is suitable for detecting selected particles (301) and a part is suitable for neutralizing selected particles (301).

11. The method according to any of claims 6 - 10, **characterized in that**, as a function of the detected particles (301), an active agent (202) suitable for neutralizing those particles (301) is brought to bind to the carrier particles (201).

12. The method according to any of claims 6 - 11, **characterized in that** a concentration of selected particles (301) in the room air is determined by the sensor (80) and an amount of active agent (202) is administered as a function of the detected concentration of particles (301).

13. The method according to any of claims 6 - 12, **characterized in that** a mixture (204) consisting of an active agent (202) bound to carrier particles (201) is produced by means of a method in which
- a mixture (200) consisting of carrier particles (201) and a liquid carrier agent is formed, which mixture is sprayed onto a hot surface (50) in a process chamber (60), where the liquid carrier agent is vaporized so as to release the carrier particles (201) into the air of the process chamber (60),
- inlet air is fed into the process chamber (60) at a selected temperature in order to cool the free carrier particles (201) to a desired temperature and form a flow of carrier air from an inlet port (15) to an outlet port (110) of the process chamber (60),
- an active agent (202) is sprayed into the process chamber (60),
- the active agent (202) is brought to collide with the free carrier particles (201) in order to bind the active agent (202) to the carrier particles (201),
- a mixture (204) consisting of active agent (202) bound to carrier particles (201) is conducted into the room air.

14. The method according to any of claims 1 - 13, **characterized in that** the particle (301) detected by the sensor (80) is identified or recorded as an unknown particle.

15. The method according to any of claims 1 - 14, **characterized in that** the carrier particle (201) is fullerene.

16. The method according to any of claims 1 - 14, **characterized in that** the carrier particle (201) is activated carbon.

17. The method according to any of claims 1 - 16, **characterized in that** the active agent (202) is honey or a product processed from honey, such as a concentrate.

18. The method according to any of claims 1 - 17, **characterized in that** the particle (301) to be detected is mould and/or Actinomycetales.

19. A system for detecting particles (301) in the air, which system is configured to produce airborne carrier particles (201), which system includes
- an air-circulating dispenser (8) configured to administer the airborne carrier particles (201) into room air,
- a sensor (80) for detecting particles (301) bound to the airborne carrier particles (201), and
- software means for storing the detection data of the sensor (80) in a memory for further processing,
**characterized in that** the dispenser (8) includes
- a process chamber (60),
- a first dispensing reservoir (91) for dispensing a mixture consisting of carrier particles (201) and a liquid carrier agent (200),
- a first feed pipe (81) for feeding the mixture of carrier particles (201) and liquid carrier agent (200) from the first dispensing reservoir (91) into the process chamber (60),
- a first spraying unit (131) comprising a nozzle for spraying the mixture (200) of carrier particles (201) and liquid carrier agent from the first dispensing reservoir (91) into a process chamber (60) via the first feed pipe (81),
- a hot surface (50) in the process chamber (60) for vaporizing the mixture (200) of carrier particles (201) and liquid carrier agent,
- an outlet port (110) for conducting the carrier particles (201) into the room air,
- an air inlet unit (10) comprising a motor (12) and a heater (13) for forming a flow of carrier air into the process chamber (60) at a selected temperature, wherein the air inlet unit (10) is connected to the process chamber (60) by an inlet port (15), and wherein the carrier air flow forms from the inlet port (15) to the outlet port (110), wherein, in the dispenser (8)
- the nozzle of the first spraying unit (131) is oriented in the process chamber (60) towards the hot surface (50) in order to spray the mixture of carrier particles (201) and liquid carrier agent onto the hot surface (50).

20. The system according to claim 19, **characterized in that** the sensor (80) is arranged in a air inlet unit (10) of the dispenser (8) or in an air outlet vent (150) of the room.

21. The system according to claim 19 or 20, **characterized in that** the sensor (80) is a photoacoustic laser.

22. The system according to any of claims 19 - 21, **characterized in that** the dispenser (8) is configured to administer an active agent (202) bound to the airborne carrier particles (201).

23. The system according to claim 22, **characterized in that** the dispenser (8) includes
- a process chamber (60),
- a first dispensing reservoir (91) for dispensing a mixture consisting of carrier particles (201) and a liquid carrier agent (200),
- a second dispensing reservoir (92) for dispensing an active agent (202),
- a first feed pipe (81) for feeding the mixture of carrier particles (201) and liquid carrier agent (200) from the first dispensing reservoir (91) into the process chamber (60),
- a second feed pipe (82) for feeding the active agent (202) from the second dispensing reservoir (92) into the process chamber (60),
- a first spraying unit (131) comprising a nozzle for spraying the mixture of carrier particles (201) and liquid carrier agent (200) from the first dispensing reservoir (91) into the process chamber (60) via the first feed pipe (81),
- a second spraying unit (132) comprising a nozzle for spraying the active agent (202) from the second dispensing reservoir (92) into the process chamber (60) via the second feed pipe (82),
- a hot surface (50) in the process chamber (60) for vaporizing the mixture (200) of carrier particles (201) and liquid carrier agent,
- an outlet port (110) for conducting a mixture (204) consisting of active agent (202) bound to carrier particles (201) into the room air,
- an air inlet unit (10) comprising a motor (12) and a heater (13) for forming a flow of carrier air into the process chamber (60) at a selected temperature, wherein the air inlet unit (10) is connected to the process chamber (60) by an inlet port (15), and wherein the carrier air flow is formed from the inlet port (15) to the outlet port (110), wherein, in the dispenser (8)
- the nozzle of the first spraying unit (131) is oriented in the process chamber (60) towards the hot surface (50) in order to spray the mixture (200) of carrier particles (201) and liquid carrier agent onto the hot surface (50),
- the nozzle of the first spraying unit (131) is closer to the hot surface (50) and to the inlet port (15) than the nozzle of the second spraying unit (132), and
- the nozzle of the second spraying unit (132) is closer to the outlet port (110) than the nozzle of the first spraying unit (131).

## Patentansprüche

1. Verfahren zur Detektion von Partikeln (301) in Raumluft, bei dem
- in der Luft befindliche Trägerpartikel (201) erzeugt werden,
- die Trägerpartikel (201) in die zirkulierende Raumluft vermittelt werden,
- die Trägerpartikel (201) mit den Partikeln (301) in der Raumluft zur Kollision gebracht werden,
- die Partikel (301) in der Raumluft an die Trägerpartikel (201) gebunden werden, wodurch sich die Suspensionseigenschaften der Partikel (301) ändern, sobald die Partikel (301) an die Trägerpartikel (201) gebunden sind,
- die Partikel (301) an die Trägerpartikel (201) gebunden werden zusammen mit der im Raum zirkulierenden Luft zu einem Sensor (80) geleitet,
- die Partikel (301) durch den Sensor (80) detektiert werden, und
- die Detektionsdaten des Sensors (80) zur weiteren Verarbeitung in einem Speicher gespeichert werden,
**dadurch gekennzeichnet, dass** die in der Luft befindlichen Trägerpartikel (201) mittels eines Verfahrens erzeugt werden, bei dem
- ein Gemisch (200) bestehend aus Trägerpartikeln (201) und einem flüssigen Trägermittel gebildet wird, das auf eine heiße Oberfläche (50) in einer Prozesskammer (60) gesprüht wird, wobei der flüssige Träger verdampft wird, um die Trägerpartikel (201) in die Luft der Prozesskammer (60) freizusetzen,
- der Prozesskammer (60) Zuluft mit einer ausgewählten Temperatur zugeführt wird, um die freien Trägerpartikel (201) auf eine gewünschte Temperatur abzukühlen und einen Trägerluftstrom von einer Einlassöffnung (15) zur Auslassöffnung (110) der Prozesskammer (60) zu bilden,
- die Trägerpartikel (201) in die Raumluft geleitet werden.

2. Verfahren nach Anspruch 1, **dadurch gekennzeichnet, dass** die Trägerpartikel (201) die Form einer Substanz haben, die auch eine neutralisierende Wirkung auf die zu detektierenden Partikel (301) aufweist.

3. Verfahren nach Anspruch 1 oder 2, **dadurch gekennzeichnet, dass** die Trägerpartikel (201) durch einen Luftzirkulationsspender (8) erzeugt und in die Raumluft vermittelt werden, wodurch die Trägerpartikel vorteilhaft in den oberen Teil des Raumes vermittelt werden.

4. Verfahren nach einem der Ansprüche 1 bis 3, **dadurch gekennzeichnet, dass** der Sensor (80) in einer Lufteinlasseinheit (10) des Spenders (8) in einem abgesetzten Teil des Raumes angeordnet ist.

5. Verfahren nach einem der Ansprüche 1 bis 3, **dadurch gekennzeichnet, dass** der Sensor (80) in einer Luftauslassöffnung (150) oder in einem Luftauslasskanal des Raumes angeordnet ist.

6. Verfahren nach einem der Ansprüche 1 bis 5, **dadurch gekennzeichnet, dass** ein Wirkstoff (202) dazu gebracht wird, sich an die in der Luft befindlichen Trägerpartikel (201) zu binden, und dass ein Gemisch (204), bestehend aus dem an die Trägerpartikel (201) gebundenen Wirkstoff (202), in die Raumluft vermittelt wird.

7. Verfahren nach Anspruch 6, **dadurch gekennzeichnet, dass** der Wirkstoff (202) die Form einer Substanz hat, die ausgewählte Partikel (301) in der Raumluft neutralisiert.

8. Verfahren nach Anspruch 6 oder 7, **dadurch gekennzeichnet, dass** der Wirkstoff (202) ein Markierungsmittel ist, dessen Eigenschaften sich in der Reaktion mit dem zu detektierenden Partikel (301) verändern, und dass das Partikel (301) über die Reaktion des Markierungsmittels detektiert wird.

9. Verfahren nach einem der Ansprüche 6 bis 8, **dadurch gekennzeichnet, dass** sich die optischen Eigenschaften des Wirkstoffs (202) bei der Reaktion mit dem zu detektierenden Partikel (301) verändern.

10. Verfahren nach einem der Ansprüche 6 bis 9, **dadurch gekennzeichnet, dass** zwei oder mehr verschiedene Wirkstoffe (202) der Raumluft zugeführt werden, wobei ein Teil zur Detektion ausgewählter Partikel (301) und ein Teil zur Neutralisierung ausgewählter Partikel (301) geeignet ist.

11. Verfahren nach einem der Ansprüche 6 bis 10, **dadurch gekennzeichnet, dass** in Abhängigkeit von den detektierten Partikeln (301) ein Wirkstoff (202), der zur Neutralisierung dieser Partikel (301) geeignet ist, dazu gebracht wird, sich an die Trägerpartikel (201) zu binden.

12. Verfahren nach einem der Ansprüche 6 bis 11, **dadurch gekennzeichnet, dass** eine Konzentration ausgewählter Partikel (301) in der Raumluft durch den Sensor (80) bestimmt wird und eine Menge des Wirkstoffs (202) in Abhängigkeit von der detektierten Konzentration der Partikel (301) zugeführt wird.

13. Verfahren nach einem der Ansprüche 6 bis 12, **dadurch gekennzeichnet, dass** ein Gemisch (204), bestehend aus einem an Trägerpartikel (201) gebundenen Wirkstoff (202), mittels eines Verfahrens erzeugt wird, bei dem
- ein Gemisch (200) bestehend aus Trägerpartikeln (201) und einem flüssigen Trägermittel gebildet wird, das auf eine heiße Oberfläche (50) in einer Prozesskammer (60) gesprüht wird, wo das flüssige Trägermittel verdampft wird, um die Trägerpartikel (201) in die Luft der Prozesskammer (60) freizusetzen,
- der Prozesskammer (60) Zuluft mit einer ausgewählten Temperatur zugeführt wird, um die freien Trägerpartikel (201) auf eine gewünschte Temperatur abzukühlen und einen Trägerluftstrom von einer Einlassöffnung (15) zur Auslassöffnung (110) der Prozesskammer (60) zu bilden,
- ein Wirkstoff (202) in die Prozesskammer (60) gesprüht wird,
- das Wirkstoff (202) mit den freien Trägerpartikeln (201) zur Kollision gebracht wird, um den Wirkstoff (202) an die Trägerpartikel (201) zu binden,
- ein Gemisch (204) bestehend aus dem an die Trägerpartikel (201) gebundenen Wirkstoff (202) in die Raumluft geleitet wird.

14. Verfahren nach einem der Ansprüche 1 bis 13, **dadurch gekennzeichnet, dass** der vom Sensor (80) detektierte Partikel (301) als unbekannter Partikel identifiziert oder aufgezeichnet wird.

15. Verfahren nach einem der Ansprüche 1 bis 14, **dadurch gekennzeichnet, dass** das Trägerpartikel (201) Fulleren ist.

16. Verfahren nach einem der Ansprüche 1 bis 14, **dadurch gekennzeichnet, dass** das Trägerpartikel (201) Aktivkohle ist.

17. Verfahren nach einem der Ansprüche 1 bis 16, **dadurch gekennzeichnet, dass** der Wirkstoff (202) Honig oder ein aus Honig verarbeitetes Produkt, wie beispielsweise ein Konzentrat, ist.

18. Verfahren nach einem der Ansprüche 1 bis 17, **dadurch gekennzeichnet, dass** das zu detektierende Partikel (301) Schimmel und/oder Actinomycetales ist.

19. System zur Detektion von Partikeln (301) in der Luft, wobei das System so konfiguriert ist, dass es in der Luft befindliche Trägerpartikel (201) erzeugt, wobei das System einschließt
- einen Luftzirkulationsspender (8), der so konfiguriert ist, dass er die in der Luft befindlichen Trägerpartikel (201) in die Raumluft abgibt,
- einen Sensor (80) zur Detektion von Partikeln (301), die an die in der Luft befindlichen Trägerpartikel (201) gebunden sind, und
- Softwaremittel zum Speichern der Detektionsdaten des Sensors (80) in einem Speicher zur ferner Verarbeitung,
**dadurch gekennzeichnet, dass** der Spender (8) einschließt
- eine Prozesskammer (60),
- einen ersten Abgabebehälter (91) zum Abgeben eines Gemisches bestehend aus Trägerpartikeln (201) und einem flüssigen Trägermittel (200),
- eine erste Zuführleitung (81) zum Füttern des Gemisches bestehend aus Trägerpartikeln (201) und flüssigem Trägermittel aus dem ersten Abgabebehälter (91) in die Prozesskammer (60),
- eine erste Sprüheinheit (131), umfassend eine Düse zum Versprühen des Gemisches (200) aus Trägerpartikeln (201) und flüssigem Trägermittel (200) aus dem ersten Abgabebehälter (91) über die erste Zuführleitung (81) in eine Prozesskammer (60),
- eine heiße Oberfläche (50) in der Prozesskammer (60) zum Verdampfen des Gemisches (200) aus Trägerpartikeln (201) und flüssigem Trägermittel,
- eine Auslassöffnung (110) zum Leiten der Trägerpartikel (201) in die Raumluft,
- eine Lufteinlasseinheit (10), umfassend einen Motor (12) und eine Heizvorrichtung (13) zum Erzeugen eines Trägerluftstroms in die Prozesskammer (60) bei einer ausgewählten Temperatur, wobei die Lufteinlasseinheit (10) über eine Einlassöffnung (15) mit der Prozesskammer (60) verbunden ist, und wobei der Trägerluftstrom von der Einlassöffnung (15) zur Auslassöffnung (110) strömt,
wobei in dem Spender (8)
- die Düse der ersten Sprüheinheit (131) in der Prozesskammer (60) auf die heiße Oberfläche (50) ausgerichtet ist, um das Gemisch aus Trägerpartikeln (201) und flüssigem Trägermittel auf die heiße Oberfläche (50) zu sprühen.

20. System nach Anspruch 19, **dadurch gekennzeichnet, dass** der Sensor (80) in einer Lufteinlasseinheit (10) des Spenders (8) oder in einer Luftauslassöffnung (150) des Raums angeordnet ist.

21. System nach Anspruch 19 oder 20, **dadurch gekennzeichnet, dass** der Sensor (80) ein photoakustischer Laser ist.

22. System nach einem der Ansprüche 19 bis 21, **dadurch gekennzeichnet, dass** der Spender (8) so konfiguriert ist, dass er ein Wirkstoff (202) abgibt, das an die in der Luft befindlichen Trägerpartikel (201) gebunden ist.

23. System nach Anspruch 22, **dadurch gekennzeichnet, dass** der Spender (8) einschließt
- eine Prozesskammer (60),
- einen ersten Abgabebehälter (91) zum Abgeben eines Gemisches bestehend aus Trägerpartikeln (201) und einem flüssigen Trägermittel (200),
- einen zweiten Spenderbehälter (92) zum Abgeben eines Wirkstoffs (202),
- eine erste Zuführleitung (81) zum Füttern des Gemisches aus Trägerpartikeln (201) und flüssigem Trägermittel (200) aus dem ersten Abgabebehälter (91) in die Prozesskammer (60),
- eine zweite Zuführleitung (82) zum Füttern des Wirkstoffs (202) aus dem zweiten Abgabebehälter (92) in die Prozesskammer (60),
- eine erste Sprüheinheit (131), die eine Düse zum Versprühen des Gemisch aus Trägerpartikeln (201) und flüssigem Trägermittel (200) aus dem ersten Abgabebehälter (91) über die erste Zuführleitung (81) in die Prozesskammer (60) umfasst,
- eine zweite Sprüheinheit (132), die eine Düse zum Versprühen des Wirkstoffs (202) aus dem zweiten Abgabebehälter (92) über die zweite Zuführleitung (82) in die Prozesskammer (60) umfasst.
- eine heiße Oberfläche (50) in der Prozesskammer (60) zum Verdampfen des Gemisches (200) aus Trägerpartikeln (201) und flüssigem Trägermittel,
- eine Auslassöffnung (110) zum Leiten eines Gemisches (204) bestehend aus an Trägerpartikeln (201) gebundenem Wirkstoff (202) in die Raumluft,
- eine Lufteinlasseinheit (10), umfassend einen Motor (12) und eine Heizvorrichtung (13) zum Erzeugen eines Trägerluftstroms in die Prozesskammer (60) mit einer ausgewählten Temperatur, wobei die Lufteinlasseinheit (10) über eine Einlassöffnung (15) mit der Prozesskammer (60) verbunden ist, und wobei der Trägerluftstrom von der Einlassöffnung (15) zur Auslassöffnung (110) gebildet wird,
wobei in dem Spender (8)
- die Düse der ersten Sprüh-Einheit (131) in der Prozesskammer (60) auf die heiße Oberfläche (50) ausgerichtet ist, um das Gemisch (200) aus Trägerpartikeln (201) und flüssigem Trägermittel auf die heiße Oberfläche (50) zu sprühen,
- die Düse der ersten Sprüheinheit (131) näher an der heißen Oberfläche (50) und an der Einlassöffnung (15) angeordnet ist als die Düse der zweiten Sprüheinheit (132), und
- die Düse der zweiten Sprüheinheit (132) näher an die Auslassöffnung (110) angeordnet ist als die Düse der ersten Sprüheinheit (131).

## Revendications

1. Procédé de détection de particules (301) dans de l'air ambiant, dans lequel
- des particules porteuses en suspension dans l'air (201) sont produites,
- les particules porteuses (201) sont acheminées dans l'air ambiant en circulation,
- les particules porteuses (201) sont amenées à entrer en collision avec les particules (301) présentes dans l'air ambiant,
- les particules (301) présentes dans l'air ambiant sont amenées à se fixer sur les particules porteuses (201), les caractéristiques de suspension des particules (301) étant modifiées dès que les particules (301) se fixent sur les particules porteuses (201),
- les particules (301), qui sont fixées sur les particules porteuses (201), sont acheminées vers un capteur (80) avec l'air circulant dans la pièce,
- les particules (301) sont détectées par le capteur (80), et
- les données de détection du capteur (80) sont stockées dans une mémoire en vue d'un traitement ultérieur,
**caractérisé en ce que** les particules porteuses en suspension dans l'air (201) sont produites au moyen d'un procédé dans lequel
- un mélange (200) composé de particules porteuses (201) et d'un agent porteur liquide est formé et pulvérisé sur une surface chaude (50) dans une chambre de traitement (60), où l'agent porteur liquide est vaporisé de manière à libérer les particules porteuses (201) dans l'air de la chambre de traitement (60),
- de l'air entrant est introduit dans la chambre de traitement (60) à une température sélectionnée afin de refroidir les particules porteuses libres (201) pour qu'elles atteignent une température souhaitée et forment un flux d'air porteur entre un orifice d'entrée (15) et un orifice de sortie (110) dans la chambre de traitement (60),
- les particules porteuses (201) sont acheminées dans l'air ambiant.

2. Procédé conformément à la revendication 1, **caractérisé en ce que** les particules porteuses (201) se présentent sous la forme d'une substance qui a également un effet neutralisant sur les particules (301) à détecter.

3. Procédé conformément à la revendication 1 ou 2, **caractérisé en ce que** les particules porteuses (201) sont produites et acheminées dans l'air ambiant par un distributeur à circulation d'air (8) au moyen duquel les particules porteuses sont avantageusement acheminées dans la partie supérieure de la pièce.

4. Procédé conformément à l'une quelconque des revendications 1 à 3, **caractérisé en ce que** le capteur (80) est placé dans une unité d'entrée d'air (10) du distributeur (8) dans une partie inférieure de la pièce.

5. Procédé conformément à l'une quelconque des revendications 1 à 3, **caractérisé en ce que** le capteur (80) est placé dans un évent de sortie d'air (150) ou dans un conduit de sortie d'air de la pièce.

6. Procédé conformément à l'une quelconque des revendications 1 à 5, **caractérisé en ce qu'**un agent actif (202) est amené à se fixer aux particules porteuses (201) en suspension dans l'air, et qu'un mélange (204) composé d'un agent actif (202) fixé aux particules porteuses (201) est acheminé dans l'air ambiant.

7. Procédé conformément à la revendication 6, **caractérisé en ce que** l'agent actif (202) se présente sous la forme d'une substance qui neutralise certaines particules (301) de l'air ambiant.

8. Procédé conformément à la revendication 6 ou 7, **caractérisé en ce que** l'agent actif (202) est un agent de marquage dont les caractéristiques sont modifiées lors de la réaction avec la particule (301) à détecter, et **en ce que** la particule (301) est détectée par la réaction de l'agent de marquage.

9. Procédé conformément à l'une quelconque des revendications 6 à 8, **caractérisé en ce que** les caractéristiques optiques de l'agent actif (202) sont modifiées lors de la réaction avec la particule (301) à détecter.

10. Procédé conformément à l'une quelconque des revendications 6 à 9, **caractérisé en ce que** deux agents actifs différents (202) ou plus sont envoyés dans l'air ambiant, où une partie permet la détection de certaines particules (301) et une autre permet la neutralisation de certaines particules (301).

11. Procédé conformément à l'une quelconque des revendications 6 à 10, **caractérisé en ce que**, en fonction des particules détectées (301), un agent actif (202) permettant de neutraliser ces particules (301) est amené à se fixer aux particules porteuses (201).

12. Procédé conformément à l'une quelconque des revendications 6 à 11, **caractérisé en ce que** la concentration de certaines particules (301) dans l'air ambiant est déterminée par le capteur (80) et une quantité d'agent actif (202) est administrée en fonction de la concentration détectée en particules (301).

13. Procédé conformément à l'une quelconque des revendications 6 à 12, **caractérisé en ce qu'**un mélange (204) composé d'un agent actif (202) fixé à des particules porteuses (201) est produit au moyen d'un procédé dans lequel
- un mélange (200) composé de particules porteuses (201) et d'un agent porteur liquide est formé et pulvérisé sur une surface chaude (50) dans une chambre de traitement (60), l'agent porteur liquide étant vaporisé de manière à libérer les particules porteuses (201) dans l'air de la chambre de traitement (60),
- de l'air entrant est introduit dans la chambre de traitement (60) à une température sélectionnée afin de refroidir les particules porteuses libres (201) pour qu'elles atteignent une température souhaitée et forment un flux d'air porteur entre un orifice d'entrée (15) et un orifice de sortie (110) dans la chambre de traitement (60),
- un agent actif (202) est pulvérisé dans la chambre de traitement (60),
- l'agent actif (202) est amené à entrer en collision avec les particules porteuses libres (201) afin de fixer l'agent actif (202) aux particules porteuses (201),
- un mélange (204) composé de l'agent actif (202) fixé aux particules porteuses (201) est acheminé dans l'air ambiant.

14. Procédé conformément à l'une quelconque des revendications 1 à 13, **caractérisé en ce que** la particule (301) détectée par le capteur (80) est identifiée ou enregistrée comme une particule inconnue.

15. Procédé conformément à l'une quelconque des revendications 1 à 14, **caractérisé en ce que** la particule porteuse (201) est un fullerène.

16. Procédé conformément à l'une quelconque des revendications 1 à 14, **caractérisé en ce que** la particule porteuse (201) est du charbon actif.

17. Procédé conformément à l'une quelconque des revendications 1 à 16, **caractérisé en ce que** l'agent actif (202) est du miel ou un produit transformé obtenu à partir de miel, tel qu'un concentré.

18. Procédé conformément à l'une quelconque des revendications 1 à 17, **caractérisé en ce que** la particule (301) à détecter est une moisissure et/ou des actinomycétales.

19. Système de détection de particules (301) dans l'air, configuré pour produire des particules porteuses en suspension dans l'air (201), comprenant
- un distributeur à circulation d'air (8) configuré pour envoyer les particules porteuses en suspension dans l'air (201) dans l'air ambiant,
- un capteur (80) pour détecter les particules (301) fixées aux particules porteuses en suspension dans l'air (201), et
- des moyens logiciels pour stocker les données de détection du capteur (80) dans une mémoire en vue d'un traitement ultérieur,
**caractérisé en ce que** le distributeur (8) comprend
- une chambre de traitement (60),
- un premier réservoir de distribution (91) pour distribuer un mélange composé de particules porteuses (201) et d'un agent porteur liquide (200),
- un premier tuyau d'alimentation (81) pour acheminer le mélange de particules porteuses (201) et d'agent porteur liquide du premier réservoir de distribution (91) à la chambre de traitement (60),
- une première unité de pulvérisation (131) comprenant une buse pour pulvériser le mélange (200) de particules porteuses (201) et d'agent porteur liquide (200) du premier réservoir de distribution (91) dans une chambre de traitement (60) via le premier tuyau d'alimentation (81),
- une surface chaude (50) dans la chambre de traitement (60) pour vaporiser le mélange (200) de particules porteuses (201) et d'agent porteur liquide,
- un orifice de sortie (110) pour acheminer les particules porteuses (201) dans l'air ambiant,
- une unité d'entrée d'air (10) comprenant un moteur (12) et un réchauffeur (13) pour la formation d'un flux d'air porteur dans la chambre de traitement (60) à une température sélectionnée, l'unité d'entrée d'air (10) étant reliée à la chambre de traitement (60) par un orifice d'entrée (15), et où le flux d'air porteur se forme entre l'orifice d'entrée (15) et l'orifice de sortie (110),
dans lequel, dans le distributeur (8)
- la buse de la première unité de pulvérisation (131) est orientée dans la chambre de traitement (60) vers la surface chaude (50) afin de pulvériser le mélange de particules porteuses (201) et d'agent porteur liquide sur la surface chaude (50).

20. Système conformément à la revendication 19, **caractérisé en ce que** le capteur (80) est placé dans une unité d'entrée d'air (10) du distributeur (8) ou dans un évent de sortie d'air (150) de la pièce.

21. Système conformément à la revendication 19 ou 20, **caractérisé en ce que** le capteur (80) est un laser photo-acoustique.

22. Système conformément à l'une quelconque des revendications 19 à 21, **caractérisé en ce que** le distributeur (8) est configuré pour envoyer un agent actif (202) fixé aux particules porteuses en suspension dans l'air (201).

23. Système conformément à la revendication 22, **caractérisé en ce que** le distributeur (8) comprend
- une chambre de traitement (60),
- un premier réservoir de distribution (91) pour distribuer un mélange composé de particules porteuses (201) et d'un agent porteur liquide (200),
- un deuxième réservoir de distribution (92) pour distribuer un agent actif (202),
- un premier tuyau d'alimentation (81) pour acheminer le mélange de particules porteuses (201) et d'agent porteur liquide (200) du premier réservoir de distribution (91) dans la chambre de traitement (60),
- un deuxième tuyau d'alimentation (82) pour acheminer l'agent actif (202) du deuxième réservoir de distribution (92) dans la chambre de traitement (60),
- une première unité de pulvérisation (131) comprenant une buse pour pulvériser le mélange de particules porteuses (201) et d'agent porteur liquide (200) du premier réservoir de distribution (91) dans la chambre de traitement (60) via le premier tuyau d'alimentation (81),
- une deuxième unité de pulvérisation (132) comprenant une buse pour pulvériser l'agent actif (202) du deuxième réservoir de distribution (92) dans la chambre de traitement (60) via le deuxième tuyau d'alimentation (82),
- une surface chaude (50) dans la chambre de traitement (60) pour vaporiser le mélange (200) de particules porteuses (201) et d'agent porteur liquide,
- un orifice de sortie (110) pour acheminer dans l'air ambiant un mélange (204) composé de l'agent actif (202) fixé à des particules porteuses (201),
- une unité d'entrée d'air (10) comprenant un moteur (12) et un réchauffeur (13) pour la formation d'un flux d'air porteur dans la chambre de traitement (60) à une température sélectionnée, l'unité d'entrée d'air (10) étant reliée à la chambre de traitement (60) par un orifice d'entrée (15), et où le flux d'air porteur est formé entre l'orifice d'entrée (15) et l'orifice de sortie (110),
dans lequel, dans le distributeur (8)
- la buse de la première unité de pulvérisation (131) est orientée dans la chambre de traitement (60) vers la surface chaude (50) afin de pulvériser le mélange (200) de particules porteuses (201) et d'agent porteur liquide sur la surface chaude (50),
- la buse de la première unité de pulvérisation (131) est plus proche de la surface chaude (50) et de l'orifice d'entrée (15) que la buse de la deuxième unité de pulvérisation (132), et
- la buse de la deuxième unité de pulvérisation (132) est plus proche de l'orifice de sortie (110) que la buse de la première unité de pulvérisation (131).
